# EUROPEAN PATENT SPECIFICATION

(11) **EP 1 926 453 B1**
(45) Date of publication and mention of the grant of the patent: **17.11.2010**
(21) Application number: 06793644.3
(22) Date of filing: 19.09.2006
(51) Int. Cl.: A61F 2/06, A61M 29/02

(54) **Catheter for Placement of a Stent**
Katheter zum Platzieren eines Stents
Cathèter pour le placement d'un stent

(30) Priority: 19.09.2005 US 718913 P
(43) Date of publication of application: 04.06.2008
(73) Proprietor: MINVASYS, 92230 Gennevilliers (FR)
(72) Inventor: VAN DER LEEST, Machiel, F-75005 Paris (FR); HILAIRE, Pierre, F-78160 Marly Le Roi (FR); MOULIN, Philippe, F-95130 Le Plessis-Bouchard (FR); LEARY, James, St Louis, MO 63105 (US)
(74) Representative: Hubert, Philippe
(86) International application number: PCT/EP2006/066509
(87) International publication number: WO 2007/033963

(56) References cited:
- EP-A2- 0 699 451
- US-A- 6 156 005
- US-B1- 6 602 226

## Description

### FIELD OF THE INVENTION

The present invention relates generally to catheter based treatments for vascular disease: More particularly, it relates to an improved apparatus which can be used in methods for performing angioplasty and stenting utilizing embolic protection to capture any potential embolic debris. The apparatus is particularly applicable for treatment of vascular disease at a carotid bifurcation.

### BACKGROUND OF THE INVENTION

Catheter based treatments, including angioplasty and stenting, represent a tremendous advancement in the treatment of obstructive vascular disease. Percutaneous transluminal angioplasty (PTA) of stenotic lesions in peripheral arteries using a balloon dilatation catheter was first reported by Gruentzig et al in 1974 (Percutaneous recanalization after chronic arterial occlusion with a new dilator-catheter modification of the Dotter technique; Dtsch Med Wochenschr 1974 Dec 6;99(49):2502-10, 2511). The first cases of percutaneous transluminal angioplasty of coronary arteries (PTCA) in humans were reported by Gruentzig et al in 1978 (Percutaneous transluminal dilatation of chronic coronary stenosis; First experiences, Schweiz Med Wochenschr 1978 Nov 4;108(44):1721-3). (See also Gruentzig et al, U.S. Patent 4,195,637, Catheter arrangement, method of catheterization, and method of manufacturing a dilatation element.) The use of a self-expanding vascular stent or endovascular prosthesis to prevent acute reclosure after coronary angioplasty in humans was reported by Sigwart et al. in 1987 (Intravascular stents to prevent occlusion and restenosis after transluminal angioplasty; N Engl J Med 1987 Mar 19;316(12):701-6). The first angioplasty of the carotid artery in humans was reported by Kerber et al in 1980 (Catheter dilatation of proximal carotid stenosis during distal bifurcation endarterectomy; Am J Neuroradiol 1980;1:348-9). Multiple centers reported results for stent-supported angioplasty of the carotid artery beginning in 1996 (Yadav et al, Angioplasty and stenting for restenosis after carotid endarterectomy. Initial experience. Stroke 1996;27:2075-2079; Wholey et al, Percutaneous transluminal angioplasty and stents in the treatment of extracranial circulation. J Invasive Cardiol 1996;9:225-31; Dorros, Carotid arterial obliterative disease: Should endovascular revascularization (stent supported angioplasty) today supplant carotid endarterectomy. J Intervent Cardiol 1996;9:193-196; Bergeron et al, Recurrent carotid disease: will stents be an alternative to surgery? J Endovasc Surg 1996;3:76-9; 21; Amor et al, Endovascular treatment of atherosclerotic internal carotid artery stenosis. J Endovasc Surg 1997;4(Suppl 1):1-14.)

Despite this tremendous progress, problems and difficulties remain in the treatment of carotid artery disease by angioplasty and stenting. In particular, the manipulation of catheters in the carotid arteries can dislodge embolic materials, such as thrombotic material and atherosclerotic plaque, which have the potential of being carried distally by the bloodstream into the cerebral vasculature and causing ischemic damage ion the brain. (Naylor et al, Randomized study of carotid angioplasty and stenting versus carotid endarterectomy: a stopped trial. J Vasc Surg 1998;28:326-34; DeMonte et al, Carotid transluminal angioplasty with evidence of distal embolisation. J Neurosurg 1989; 70:138-41.)

Methods and devices for embolic protection have been devised to reduce the potential risks of embolization and ischemic damage during carotid angioplasty (Theron et al, New triple coaxial catheter system for carotid angioplasty with cerebral protection. AJNR 1990; 11:869-874) and during carotid stenting (Theron et al, Carotid artery stenosis: treatment with protected balloon angioplasty and stent placement. Radiology. 1996 Dec;201(3):627-36). (See also Theron, U.S. Patent 5, 423,742, Method for the widening of strictures in vessels carrying body fluid, and Theron, U.S. Patent 6,156,005 Ballon catheter for stent implantation.)

Other recent advances in stent delivery technology are described in U.S. Patent Application, serial number 10/950,179, filed on September 24, 2004, Method for protected angioplasty and stenting at a carotid bifurcation, U.S. Patent Application, serial number 10/950,180, filed on September 24, 2004, Catheter system for protected angioplasty and stenting at a carotid bifurcation, and U.S. Patent Application, serial number 10/833,494, filed on April 27, 2004, Catheter system for stenting bifurcated vessels.

Distal embolic protection devices currently available for use in performing protected angioplasty and stenting of carotid arteries include filter devices to capture potential emboli and occlusion balloon catheters combined with aspiration to remove potential emboli. The commercially available systems tend to be costly and somewhat cumbersome to use. Another disadvantage of using distal embolic protection devices is that placement of the device distal to the treatment site tends to cause a spasm of the distal cervical internal carotid artery, which can sometimes lead to serious complications. Other approaches, such as retrograde blood flow or proximal occlusion of the carotid artery, have not yet been shown to be effective at reducing embolic complications.

What is desired therefore is improved apparatus and methods for performing protected angioplasty and stenting of carotid arteries, which is simple to operate, that effectively reduces embolic complications and which is free from complications due to spasm of the distal cervical internal carotid artery.

EP-A-699 451 discloses a stent-carrying catheter with a stent held inside an outer slidable sheath and a balloon positioned distally of the stent so that the balloon can be retracted to further expand the stent when released.

### SUMMARY OF THE INVENTION

In keeping with the foregoing discussion, the present invention provides improved apparatus to be used in methods for performing angioplasty and stenting that utilize an embolic protection device combined with aspiration to capture and remove any potential embolic debris. The apparatus and methods are particularly applicable to the treatment of vascular disease at a carotid bifurcation.

The apparatus of the invention takes the form of an integrated catheter system for angioplasty and stenting with distal embolic protection and aspiration. The catheter system can be configured in a rapid-exchange version or in an over-the-wire version. The rapid-exchange version of the catheter system includes a self-expanding stent, a stent delivery sheath, a combination angioplasty balloon catheter and stent pusher catheter, an embolic protection device and an auto-releasing sheath. The over-the-wire version of the catheter system includes a self-expanding stent, a stent delivery sheath, a combination angioplasty balloon catheter and stent pusher catheter, and an embolic protection device. The embolic protection device can be configured as an embolic protection balloon catheter or an embolic protection filter catheter.

The present invention concerns a catheter system for stenting and angioplasty, as defined in claim 1 and may comprise:
a stent delivery sheath having a proximal end and a distal end and an internal lumen;
a self-expanding stent having an unexpanded condition and an expanded condition;
and a combination angioplasty and stent pusher catheter having a catheter shaft with an expandable member mounted near a distal end of said catheter shaft;
wherein said catheter system has an undeployed configuration in which said self-expanding stent is in said unexpanded condition and is positioned within a distal portion of said internal lumen of said stent delivery sheath, and said combination angioplasty and stent pusher catheter is positioned within said internal lumen of said stent delivery sheath proximal to said self-expanding stent, whereby said combination angioplasty and stent pusher catheter can be used to deploy said self-expanding stent by retracting said stent delivery sheath while maintaining said combination angioplasty and stent pusher catheter to release said self-expanding stent out of said distal end of said stent delivery sheath thereby allowing said self-expanding stent to expand to said expanded condition, and subsequently advancing said combination angioplasty and stent pusher catheter distally until said expandable member is located within said self-expanding stent and expanding said expandable member to further expand said setf-expanding stent.

A method where the invention may be used includes steps of: inserting a guiding catheter into a target vessel in a patient's vascular system, for example at the site of a carotid bifurcation; inserting the catheter system into the guiding catheter and advancing the distal end of the catheter system to the distal end of the guiding catheter (when using the rapid exchange version of the catheter system, the auto-release sheath will automatically release itself from the catheter system during this step); advancing the embolic protection device beyond the lesion in order to support stent delivery; positioning the stent and balloon segment of the catheter system at the lesion; releasing the self-expanding stent by pulling the stent delivery sheath while maintaining the position of the combination angioplasty balloon catheter and stent pusher catheter; pulling the stent delivery sheath back into the guiding catheter; positioning and deploying the embolic protection device, preferably within the lumen of the deployed stent; advancing the combination angioplasty balloon catheter and stent pusher catheter and inflating the angioplasty balloon within the lesion; deflating the angioplasty balloon and withdrawing the combination angioplasty balloon catheter and stent pusher catheter and stent delivery sheath together; aspirating through the guiding catheter; then undeploying and withdrawing the embolic protection device to complete the procedure.

Among the three standard technical steps in the technique of carotid angioplasty and stenting, (A) prestenting angioplasty, (B) deployment of the stent, and (C) poststenting angioplasty, the most dangerous, by far, is the poststenting angioplasty step in terms of the embolic risk from detachment of cholesterol particles in the cerebral circulation. Theron et al have reported results from a series of patients confirming this and now routinely use cerebral protection only at the poststenting angioplasty step without any complication. The technical evolution in stent devices has made this possibility even more favorable because the lower profile and flexibility of most new stents allows them to be positioned without performing a prestenting angioplasty in most cases.

With the catheter system of the present invention, the embolic protection device is preferably deployed only after initial stent placement, and preferably with the occlusion balloon inflated within the lumen of the deployed stent, rather than downstream or distally from the stent. This technique has significant advantages over prior methods in that (a) inflation of the occlusion balloon inside the stent provides a full and reliable occlusion of the carotid artery; (b) inflation within the stent provides a more positive fixation of the balloon without migration of the balloon or movement of the balloon during catheter exchanges; (c) the volume to purge is significantly less than with occlusion balloons positioned more distally, which will increase the efficacy of the aspiration of potential embolic particles after angioplasty; and (d) spasm of the distal carotid artery is effectively eliminated. The configuration of the catheter system, however, allows some flexibility in this step of the method. In situations where it is preferred, the occlusion balloon can be positioned and inflated prior to deployment of the stent and/or at a position distal to the treatment site.

Preferably, the guiding catheter is introduced into the lumen of the stent after deployment of the stent. This step provides additional advantages by: (e) simplifying catheter manipulations in the subsequent steps by providing a positive pathway for advancing the catheters into the lumen of the stent; and (f) further reducing the volume that must be purged of potential emboli.

These and other advantages will be apparent upon reading the following detailed description of the invention.

### BRIEF DESCRIPTION OF THE DRAWINGS

FIG 1 is a side view of a rapid exchange version of a catheter system for protected stenting and angioplasty of a patient's carotid artery.
FIG 2 is a detail drawing of the slotted portion of the stent delivery sheath.
FIG 3 shows a cross section of a proximal section of the catheter system of FIG 1.
FIG 4 is a detail drawing showing an optional configuration of the embolic protection balloon catheter.
FIG 5 is a detail drawing showing an optional feature of the catheter system wherein the embolic protection balloon is configured to act as a catheter tip for the stent delivery sheath.
FIG 6 is a detail drawing showing an optional feature of the catheter system herein the stent delivery sheath has a floating catheter tip.
FIG 7 is a detail drawing showing an optional configuration of the proximal end of the catheter system.
FIG 8 is a side view of a coaxial over-the-wire version of a catheter system for protected stenting and angioplasty of a patient's carotid artery.
FIG 9 illustrates a patient's carotid arteries with an atherosclerotic plaque at the carotid bifurcation.
FIG 10 shows a guiding catheter positioned in the patient's common carotid artery and a guidewire advanced across the stenosis.
FIG 11 illustrates the optional step of dilating the stenosis prior to stenting with a small diameter angioplasty balloon.
FIG 12 shows the embolic protection balloon catheter advanced across the stenosis.
FIG 13 shows the stent delivery sheath advanced across the stenosis and deploying a self-expanding stent within the lesion.
FIG 14 illustrates the self-expanding stent deployed within the lesion.
FIG 15 shows the distal end of the guiding catheter advanced into the lumen of the deployed self-expanding stent and the occlusion balloon inflated within the lumen of the self-expanding stent.
FIG 16 shows the combination angioplasty balloon catheter and stent pusher catheter positioned with the angioplasty balloon across the lesion.
FIG 17 shows an angiography study performed to confirm occlusion of the internal carotid artery prior to dilatation of the lesion.
FIG 18 shows the angioplasty balloon inflated to dilate the stenosis and complete the deployment of the self-expanding stent.
FIG 19 illustrates potential embolic material being aspirated through the lumen of the guiding catheter.
FIG 20 illustrates the patient's carotid bifurcation after completion of the protected angioplasty and stenting procedure.

### DETAILED DESCRIPTION OF THE INVENTION

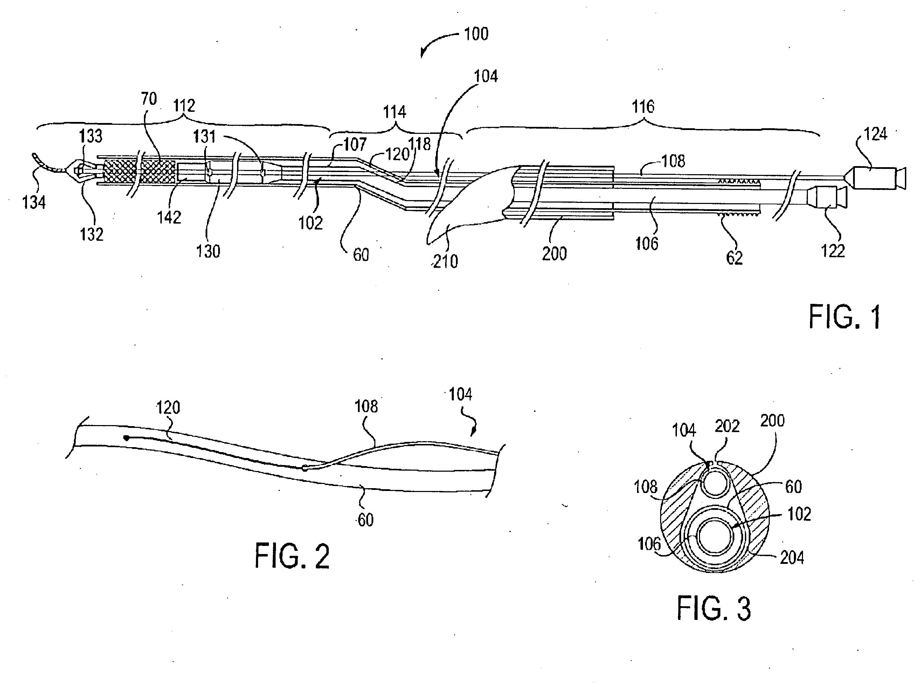

FIG 1 is a side view of a rapid exchange version of a catheter system 100 for protected stenting and angioplasty of a body passage, such as a patient's carotid artery. This version of the catheter system 100 has five major components: a self-expanding stent 70, a stent delivery sheath 60, a combination angioplasty balloon catheter and stent pusher catheter 102, an embolic protection device 104 and an auto-releasing sheath 200. The catheter system 100 has a distal section 112, a transition section 114 and a proximal section 116. The embolic protection device 104 can be configured as an embolic protection balloon catheter or an embolic protection filter catheter.

In the distal section 112, the catheter system 100 has a coaxial arrangement with the embolic protection device l04 on the inside, the combination angioplasty balloon catheter and stent pusher catheter 102 in between, and the stent delivery sheath 60 on the outside. The self-expanding stent 70 is positioned inside the stent delivery sheath 60, distal to the combination angioplasty balloon catheter and stent pusher catheter 102.

In the transition section 114 of the catheter system 100, the embolic protection device 104 passes through a first slit or hole 118 on the side of the combination angioplasty balloon catheter and stent pusher catheter 102 and a second slit or hole 120 in the side of the stent delivery sheath 60. The first slit or hole 118 in the side of the combination angioplasty balloon catheter and stent pusher catheter 102 and the second slit or hole 120 in the side of the stent delivery sheath 60 allow the embolic protection device 104, the combination angioplasty balloon catheter and stent pusher catheter 102, and the stent delivery sheath 60 to be moved independently of one another after insertion into the patient's vascular system.

In the proximal section 116 of the catheter system 100, the embolic protection device 104 is arranged side-by-side with the stent delivery sheath 60, and the combination angioplasty balloon catheter and stent pusher catheter 102 and the stent delivery sheath 60 are arranged coaxially with one another. Prior to insertion into the patient's vascular system, the auto-releasing sheath 200 is arranged on the outside of the embolic protection device 104 and the stent delivery sheath 60. The auto-releasing sheath 200 extends a substantial portion of the proximal section 116 of the catheter system 100 to hold the embolic protection device 104 and the stent delivery sheath 60 together in longitudinal alignment so that the catheter system 100 can be conveniently handled and inserted into the patient as a single unit. FIG 3 shows a cross section of a proximal section 116 of the catheter system of FIG 1.

In one preferred embodiment, the embolic protection device 104 is configured as an embolic protection balloon catheter constructed with a tubular catheter shaft 108 with an embolic protection member 132 in the form of an inflatable embolic protection balloon mounted at its distal end and a proximal connector 124, such as a luer fitting, attached at its proximal end. Preferably, a radiopaque marker 133 is located near the distal end of the tubular catheter shaft 108 to show the position of the embolic protection balloon 132 on fluoroscopy. The inflatable embolic protection balloon is preferably molded from of a highly elastic polymer, such as latex, silicone or polyurethane. An inflation lumen extends through the tubular catheter shaft from the proximal connector to an inflation port that communicates with the interior of the inflatable embolic protection balloon. The embolic protection balloon will preferably have an inflated diameter in the range of approximately 6 to 9 mm and a deflated diameter as close as practically possible to the outside diameter of the tubular catheter shaft. In a preferred embodiment of the embolic protection device 104, the tubular catheter shaft 108 is constructed, of a flexible metal tube with a diameter of 0.014 to 0.018 inches (one inch = 0.25 mm) and a length that is preferably approximately 120-170 cm or longer. The tubular catheter shaft 108 can be made, for example, from stainless steel such as 302 or 304 stainless, a cobalt alloy such as MP35 or Elgiloy, or a highly flexible or superelastic Titanium or NiTi alloy. In a preferred embodiment, a short length of coiled-wire guidewire 134 with a tapered core wire is attached to the metal tube, for example by welding, soldering, crimping, and/or adhesive. Alternatively, the flexible guidewire tip may be constructed of a resilient polymer or polymer composite with similar characteristics to a coiled-wire guidewire. This construction provides the embolic protection device 104 with highly desirable handling characteristics similar to a floppy tip steerable guidewire. Alternatively, the tubular catheter shaft can be constructed from a polymer tube or a reinforced polymer composite tube.

Optionally, the embolic protection device 104 may be constructed with a through-lumen instead of having a guidewire tip and the catheter system 100 may also include a separate steerable guidewire that extends through the lumen of the embolic protection device 104.

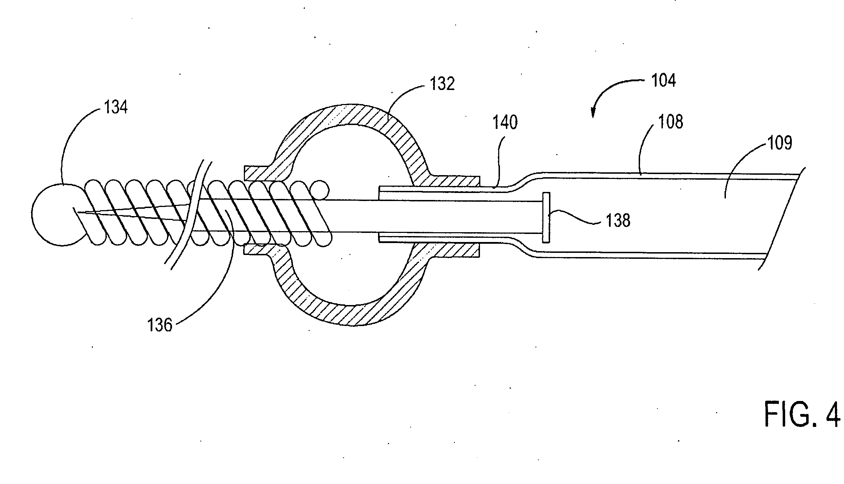

FIG 4 is a detail drawing showing an optional configuration for the distal end of the embolic protection device 104. A short length of coiled-wire guidewire 134 is attached to the distal end of a tapered core wire 136, for example by welding, soldering, crimping, and/or adhesive. Preferably, the tapered distal end of the core wire extends to the distal end of the guidewire where it is attached by welding, soldering, crimping, and/or adhesive. Optionally, the tapered core wire may have a flattened distal section to create a very flexible floppy tip. Alternatively, there may be a flexible safety wire with a flat cross section that extends to the distal end of the guidewire. The core wire 136 extends proximally of the guidewire 134 and has a proximal end with an increased diameter portion 138. The increased diameter portion 138 can be created by flattening the proximal end of the core wire. Alternatively, the increased diameter portion 138 can be created by welding to form a weld bead, or by adding material to the core wire 136 by welding, soldering or adhesive.

The tubular catheter shaft 108 is constructed of a flexible metal tube with an inflation lumen 109 extending though the tube. The increased diameter portion 138 on the proximal end of the core wire 136 is inserted into the inflation lumen 109 at the distal end of the tube. Then, the distal end of the metal tube is swaged, for example by rotary swaging, to decrease the diameter of the inflation lumen 109, effectively trapping the increased diameter portion 138 on the proximal end of the core wire 136 in the inflation lumen 109. This creates a sliding attachment between the guidewire core wire and the flexible metal tube. Swaging also creates an external shoulder 140 on the distal end of the tubular catheter shaft that provides a recessed area for attachment of the proximal sleeve of the embolic protection balloon 132 with adhesive. The distal sleeve of the embolic protection balloon is attached at the proximal end of the guidewire with adhesive. The sliding attachment of the guidewire core wire within the inflation lumen of the tubular catheter shaft allows the guidewire to move distally during balloon inflation to accommodate the expansion of the balloon. This feature reduces the stress on the attachments of the proximal and distal balloon sleeves during balloon inflation.

In an alternate embodiment, the embolic protection device 104 can be configured as an embolic protection filter catheter. In this embodiment, the embolic protection device 104 is constructed with an elongated catheter shaft 108 with an embolic protection member 132 in the form of an expandable embolic protection filter mounted near the distal end. The embolic protection filter may be self-actuating or it may be selectively actuated with an actuating mechanism operable from the proximal end of the catheter shaft 108. Other examples of embolic protection filter catheters that can be adapted for use with the present invention are described in the following patents: US5941896, US6355051, US6991641, US6755846, US6391044, US 6142987, US6887256, US6645224, US6432122, US6336934, US 6027520, US7048752, US7033375, US6989019, US6949103, US6712835., US6605102, US6506204, US6168622, US6620182, US6616679, US6589263, US6544279, US6530939, US6371970, US6348062, US6214026, US6203561, US6179861, US6129739, US6969396, US6726702, US6663651, US6361546.

The combination angioplasty balloon catheter and stent pusher catheter 102 has a construction similar to a rapid exchange angioplasty balloon catheter with a single-lumen proximal catheter shaft 106 connected to a two-lumen distal catheter shaft 107. An approximately cylindrical inflatable angioplasty balloon 130 is mounted near the distal end of the two-lumen distal catheter shaft 107. Preferably, two radiopaque markers 131 are positioned on the distal catheter shaft 107 to show the position of the angioplasty balloon 130 on fluoroscopy. A balloon inflation lumen extends from a proximal connector 122, such as a luer fitting, on the proximal end of the catheter through the single-lumen proximal catheter shaft and through most of the two-lumen distal catheter shaft where it makes a fluid connection with the interior of the inflatable angioplasty balloon. A guidewire lumen extends from the distal tip of the catheter 106 through the two-lumen distal catheter shaft and terminates at the first slit or hole 118 on the side of the catheter. The guidewire lumen is sized to have a sliding fit with the tubular catheter shaft 108 of the embolic protection device 104. The balloon inflation lumen and the guidewire lumen may be arranged coaxially or side-by-side in the two-lumen distal catheter shaft. A shoulder 142 is formed near the distal end of the combination angioplasty balloon catheter and stent pusher catheter 102. The shoulder 142 is sized and configured to have a sliding fit with the inside of the stent delivery sheath 60 and to act as a pusher for pushing the self-expanding stent 70 out the distal end of the stent delivery sheath 60.

In one preferred embodiment of the combination angioplasty balloon catheter and stent pusher catheter 102, the single-lumen proximal catheter shaft 106 is constructed of a flexible metal tube made, for example, from stainless steel such as 302 or 304 stainless, a cobalt alloy such as MP35 or Elgiloy, or a highly flexible or superelastic Titanium or NiTi alloy. Alternatively, the single-lumen proximal catheter shaft may be made from an extruded polymer tube or a reinforced polymer composite tube. The two-lumen distal catheter shaft is preferably made from an inner extruded polymer tube that forms the guidewire lumen and a coaxial outer extruded polymer tube that forms the distal portion of the balloon inflation lumen. The first slit or hole 118 is located in the transition section 114 on the side of the catheter just distal to the junction between the single-lumen proximal catheter shaft and the two-lumen distal catheter shaft. The inflatable angioplasty balloon is typically formed by blow molding of an extruded polymer tube made, for example, from polyethylene, polyolefin, polyethylene terephthalate, polyvinyl chloride, polyamide, or alloys or copolymers thereof. The angioplasty balloon is bonded to the distal catheter shaft by welding and/or adhesive.

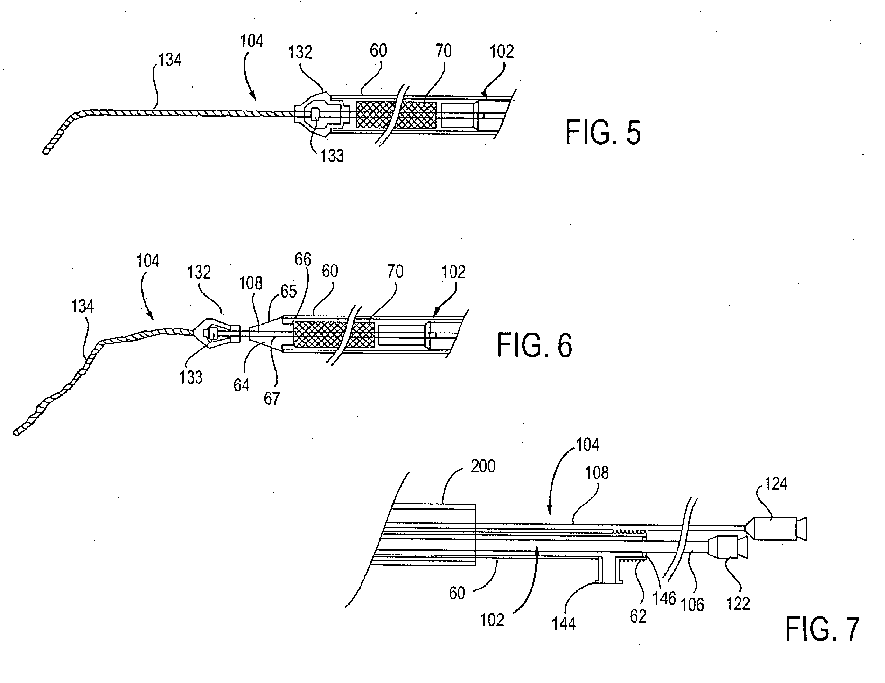

The stent delivery sheath 60 is constructed as a thin-walled single-lumen tube with an inner lumen sized to fit the self-expanding stent 70 in a compressed state. Preferably, the stent delivery sheath 60 has an external diameter as small as practically possible in order to fit through the guiding catheter and to pass through a stenosis without predilatation. Preferably, the external diameter of the stent delivery sheath 60 will be less than 6 French (approximately 2 mm diameter), more preferably, less than 5 French (approximately 1.7 mm diameter). The stent delivery sheath 60 is preferably formed of an extruded polymer tube made, for example, from polyimide, polyethylene, polypropylene, polyolefin, polyurethane, polyethylene terephthalate, polyvinyl chloride, polyamide, or alloys, copolymers or reinforced composites thereof. At the level of the transition section of the catheter system there is a second slit or hole 120 in the side of the stent delivery sheath 60. Preferably, a gripping portion 62 is formed at the proximal end of the stent delivery sheath 60 for convenience in handling. In one particularly preferred embodiment of the stent delivery sheath 60 shown in FIG 7, the proximal end of the stent delivery sheath 60 has a side port 144 with a connector, such as a luer fitting, for purging the catheter system 100 of air prior to use and, optionally, for aspirating or irrigating through the lumen of the stent delivery sheath 60 and a hemostasis valve 146 forming a sliding seal with the proximal catheter shaft 106 of the angioplasty balloon catheter and stent pusher catheter 102.

The self-expanding stent 70 is typically constructed as a braided tube of resilient wire made, for example, from stainless steel such as 302 or 304 stainless, a cobalt alloy such as MP35 or Elgiloy, or a highly flexible or superelastic Titanium or NiTi alloy. The self-expanding stent 70 will preferably have an expanded diameter of approximately 7 to 9 mm and a length that is preferably 5 cm or more to accommodate a majority of patients' carotid arteries. The self-expanding stent 70 will have a compressed diameter as small as practically possible in order to fit inside of the stent delivery sheath 60. The length of the self-expanding stent 70 in the compressed state will depend on the foreshortening of the stent when it expands. Other constructions are possible for the self-expanding stent 70. For example, a self-expanding stent 70 having a nested ring pattern or the like can be laser cut from a tube of highly resilient material, such as a highly flexible or superelastic Titanium or NiTi alloy. This construction of self-expanding stent can be configured to have little or no foreshortening of the stent when it expands.

The auto-releasing sheath 200 is preferably constructed as a single lumen extruded polymer tube with a longitudinal slit 202 along the length of the sheath. The lumen of the sheath is sized and configured to have a snug fit around the tubular shaft 108 of the embolic protection device 104 side-by-side with the stent delivery sheath 60 (with the combination angioplasty balloon catheter and stent pusher catheter 102 located coaxially inside the sheath 60). The auto-releasing sheath 200 extends a substantial portion of the proximal section 116 of the catheter system 100 to hold the embolic protection device 104 and the stent delivery sheath 60 together in longitudinal alignment so that the catheter system 100 can be conveniently handled and inserted into the patient as a single unit. The distal end of the auto-releasing sheath 200 is cut at a diagonal with a tab 210 located on the opposite side from the slit 202 to facilitate the auto-releasing of the sheath 200 during insertion of the catheter system 100 into the patient. Other possible configurations of the auto-releasing sheath 200 and other catheter linking devices usable with the catheter system 100 are described in american patent application 10/833,494.

In one particularly preferred embodiment shown in cross section in FIG 3, the auto-releasing sheath 200 is manufactured as an extruded profile with an approximately circular outer profile and an approximately oval inner lumen 204. The longitudinal split 202 connects the inner lumen 204 with the exterior of the auto-releasing sheath 200 at a thin part of the wall that coincides with the major axis of the oval inner lumen 204. The longitudinal split 202 is preferably formed during the extrusion of the auto-releasing sheath 200. Alternatively, the tube 200 can be extruded without the longitudinal split 202 and then slitted along the length to form the longitudinal split 202 in a secondary operation. The longitudinal split 202 allows the auto-releasing sheath 200 to be placed over the proximal sections 106, 108 of the catheters 102, 104 during assembly of the catheter system 100 and to be removed from the catheters 102, 104 at the appropriate time during the protected angioplasty and stenting procedure. Suitable materials for the auto-releasing sheath 200 include polyamide copolymers (e.g. PEBAX 6333 or PA 8020 from ATOFINA), polypropylene, and any extrudable medical grade polymer with a suitable combination of strength, flexibility and friction characteristics.

The auto-releasing sheath 200 has the advantage that, once it is started, the auto-releasing sheath 200 will demount itself as the catheter system 100 is advanced so that the physician does not need to unpeel, remove or displace a linking member that would otherwise require a "third hand". The catheter system 100 is prepared for use by aligning the combination angioplasty balloon catheter and stent pusher catheter 102 and the embolic protection device 104 in the desired longitudinal alignment and then pressing the longitudinal split 202 of the auto-releasing sheath 200 against the proximal sections 106, 108 of the catheters until they are enclosed within the inner lumen 204 of the auto-releasing sheath 200, as shown in FIG 3. This preparation is preferably carried out at the manufacturing facility or, alternatively, it may be performed at the point of use by a medical practitioner. The distal ends of the combination angioplasty balloon catheter and stent pusher catheter 102 and the embolic protection device 104 are inserted into the patient in the usual manner through a guiding catheter with a Y-fitting or other hemostasis adapter on the proximal end of the guiding catheter. The distal pull-tab 210 is pulled toward the side to start demounting the auto-releasing sheath 200 from the combination angioplasty balloon catheter and stent pusher catheter 102 and the embolic protection device 104, and then the catheter system 100 is advanced as a unit. When the auto-releasing sheath 200 encounters the Y-fitting, the auto-releasing sheath 200 will peel away or demount itself from the proximal sections 106, 108 of the combination angioplasty balloon catheter and stent pusher catheter 102 and the embolic protection device 104. Once the combination angioplasty balloon catheter and stent pusher catheter 102 and the embolic protection device 104 have been advanced into the distal part of the guiding catheter, the auto-releasing sheath 200 can be set aside and discarded.

FIG 5 is a detail drawing showing an optional feature of the catheter system 100 wherein the embolic protection balloon 132 is configured to act as a catheter tip for the stent delivery sheath 60. During insertion of the catheter system 100, the embolic protection balloon device 104 is withdrawn into the stent delivery sheath 60 so that the embolic protection balloon nestles into the distal end of the stent delivery sheath 60 and creates a smooth tapered distal end on the sheath 60 for passing though a stenosis in an artery without disturbing the plaque on the arterial walls.

FIG 6 is a detail drawing showing an optional feature of the catheter system 100 wherein the stent delivery sheath 60 has a floating catheter tip 64. The floating catheter tip 64 has a conical distal shape 65 and has a proximal shoulder 66 configured to nest into the distal tip of the stent delivery sheath 60. The tubular catheter shaft 108 of the embolic protection device 104 passes through a central hole 67 in the floating catheter tip 64. During insertion of the catheter system 100, the floating catheter tip 64 provides a smooth tapered distal end on the sheath 60 for passing though a stenosis in an artery without disturbing the plaque on the arterial walls. When the self-expanding stent 70 is deployed, the floating catheter tip 64 is pushed out of the lumen of the stent delivery sheath 60 to allow deployment of the stent 70. The floating catheter tip 64 is effectively trapped on the tubular catheter shaft 108 of the embolic protection device 104.

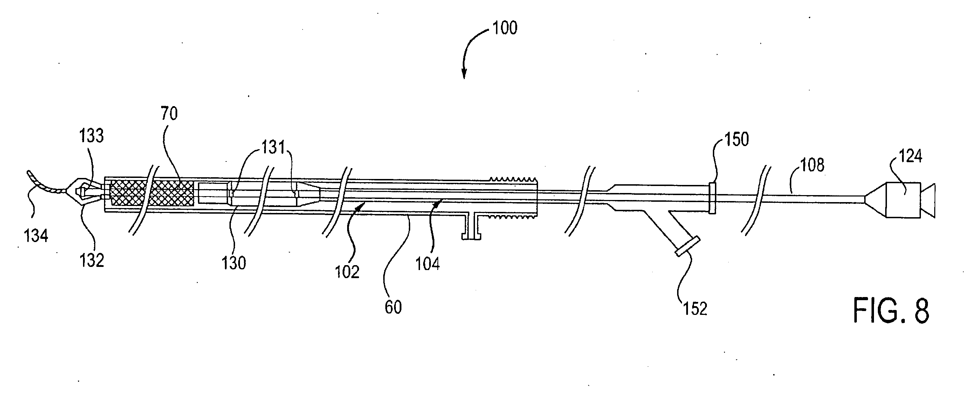

FIG 8 is a side view of a coaxial over-the-wire version of a catheter system 100 for protected stenting and angioplasty of a body passage, such as a patient's carotid artery. This version of the catheter system 100 has four major components: a self-expanding stent 70, a stent delivery sheath 60, a combination angioplasty balloon catheter and stent pusher catheter 102 and an embolic protection device 104. The embolic protection device 104 can be configured as an embolic protection balloon catheter or an embolic protection filter catheter. The catheter system 100 has a coaxial arrangement throughout the entire length, with the embolic protection device 104 on the inside, the combination angioplasty balloon catheter and stent pusher catheter 102 in between, and the stent delivery sheath 60 on the outside. The self-expanding stent 70 is positioned inside the stent delivery sheath 60, distal to the combination angioplasty balloon catheter and stent pusher catheter 102. In most respects, the components in this version of the catheter system are similar in construction to those described above. However, in this version the combination angioplasty balloon catheter and stent pusher catheter 102 is configured similar to an over-the-wire angioplasty balloon catheter with a guidewire lumen and a balloon inflation lumen that extend the full length of the catheter and which connect, respectively, to a guidewire insertion hub 150 and a balloon inflation hub 152 on the proximal end of the catheter 102. Since the components are coaxial throughout the entire length of the catheter system, there is no need for the transition section or the first and second slits or holes in the stent delivery sheath 60 and the combination angioplasty balloon catheter and stent pusher catheter 102, as in the rapid-exchange version of the catheter system described above. Also there is no need for the auto-releasing sheath 200. In this version, the tubular catheter shaft 108 of the embolic protection device 104 has a length of approximately twice the length of the stent delivery sheath 60 and the combination angioplasty balloon catheter and stent pusher catheter 102 to allow these components to be withdrawn completely out of the guiding catheter while the embolic protection member 132 remains deployed and in place during the aspiration step of the method.

Alternative configurations that allow the stent delivery sheath 60 and the combination angioplasty balloon catheter and stent pusher catheter 102 to be withdrawn completely out of the guiding catheter while the embolic protection balloon remains inflated and in place include: a removable luer hub combined with a valve within the tubular shaft of the embolic protection catheter, as described in U.S. patent 6,156,005; and a low profile catheter shaft with means for inflating the embolic protection balloon, as described in U.S. patent 6,641,573.

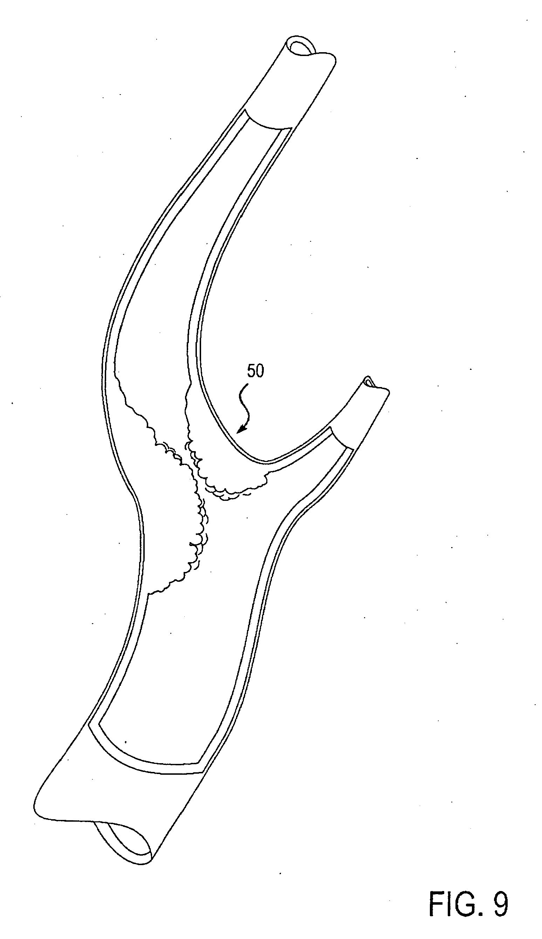

FIGS 9-20 illustrate a method for protected stenting and angioplasty according to the invention. The method includes steps of: inserting a guiding catheter into a target vessel in a patient's vascular system, for example at the site of a carotid bifurcation; inserting the catheter system into the guiding catheter and advancing the distal end of the catheter system to the distal end of the guiding catheter (when using the rapid exchange version of the catheter system, the auto-release sheath will automatically release itself from the catheter system during this step); advancing the embolic protection balloon catheter (or embolic protection filter catheter) beyond the lesion in order to support stent delivery; positioning the stent and balloon segment of the catheter system at the lesion; releasing the self-expanding stent by pulling the stent delivery sheath while maintaining, the position of the combination angioplasty balloon catheter and stent pusher catheter; pulling the stent delivery sheath back into the guiding catheter; positioning and inflating the embolic protection balloon, preferably within the lumen of the deployed stent; advancing the combination angioplasty balloon catheter and stent pusher catheter and inflating the angioplasty balloon within the lesion; deflating the angioplasty balloon and withdrawing the combination angioplasty balloon catheter and stent pusher catheter and stent delivery sheath together; aspirating through the guiding catheter; then deflating and withdrawing the embolic protection balloon catheter to complete the procedure.

FIG 9 illustrates a patient's carotid arteries with an atherosclerotic plaque 50 at the carotid bifurcation. The carotid bifurcation is a unique anatomical spot of the human body because of the carotid sinus. This dilatation at the origin of the internal carotid artery and the external carotid artery creates an area of turbulent flow that represents a kind of filter for the cerebral vasculature: the particles of cholesterol that circulate in the artery deposit on the arterial wall, mainly the posterior wall. There is usually no deposit of cholesterol above the site of the bifurcation. One of the goals of the present invention is to concentrate the whole procedure on the actual pathological area, which is limited in length and volume.

The procedure begins by establishing arterial access, typically with a needle puncture of the femoral artery or radial artery. A 7 or 8 French (one French = 0.3 mm) introducer sheath is positioned in the artery at the puncture site using a standard Seldinger technique or other known insertion technique. The common carotid artery is catheterized with a 5 French diagnostic catheter and an exchange guidewire is advanced through the diagnostic catheter into the common carotid artery.

The diagnostic catheter is withdrawn and a 7 or 8 French guiding catheter 52, with as vertebral curve or other suitable distal curve, is advanced over the exchange guidewire into the common carotid artery. The exchange guidewire is withdrawn and angiography is performed by injecting radiopaque dye through the lumen of the guiding catheter 52.

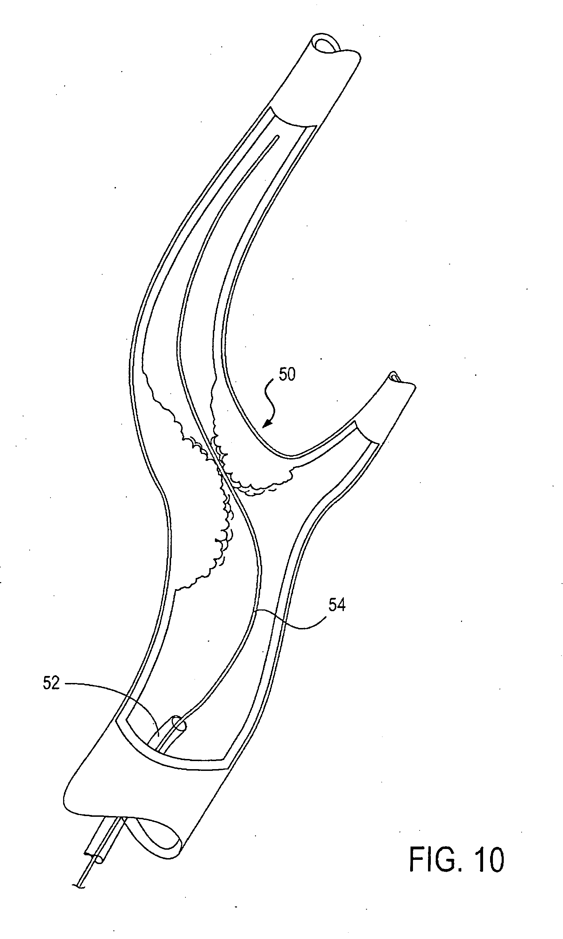

Next, a guidewire 54 is advanced through the guiding catheter 52 and across the stenosis 50 in the carotid artery. FIG 10 shows a guiding catheter 52 positioned in the patient's common carotid artery and a guidewire 54 advanced across the stenosis. Preferably, a coronary style steerable guidewire with a diameter of 0.014 to 0.018 inches is used. Alternatively, the catheter system can be modified to use other diameters of guidewire such as 0.035 to 0.038 inches, (one inch = 25.4 mm).

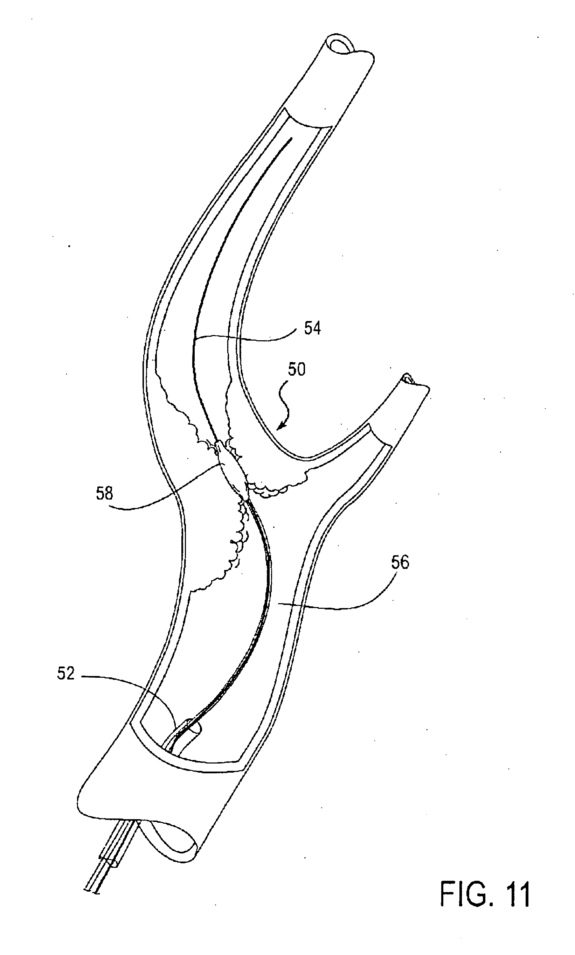

When necessary (in less than 5% of the cases), a prestenting angioplasty (typically using a rapid exchange style angioplasty catheter 56 with a 2 mm diameter dilatation balloon 58) is performed without embolic protection to facilitate stent crossing. FIG 11 illustrates this optional step of dilating the stenosis prior to stenting. After the stenosis has been dilated, the balloon 58 is deflated and the angioplasty catheter 56 and the guidewire 54 are withdrawn.

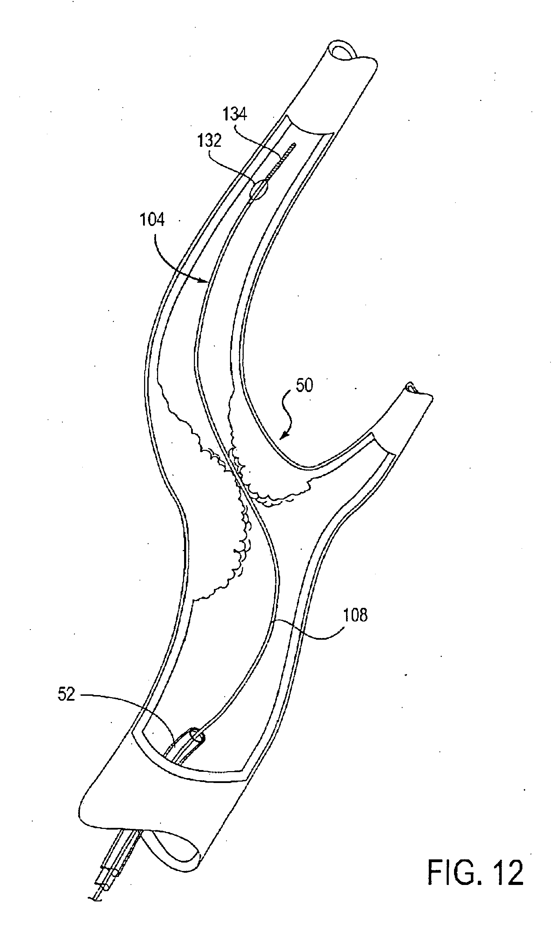

FIG 12 shows the embolic protection device 104 advanced across the stenosis 50. The tubular shaft 108 of the embolic protection device 104 serves the function of a guidewire for establishing catheter access across the stenosis 50.

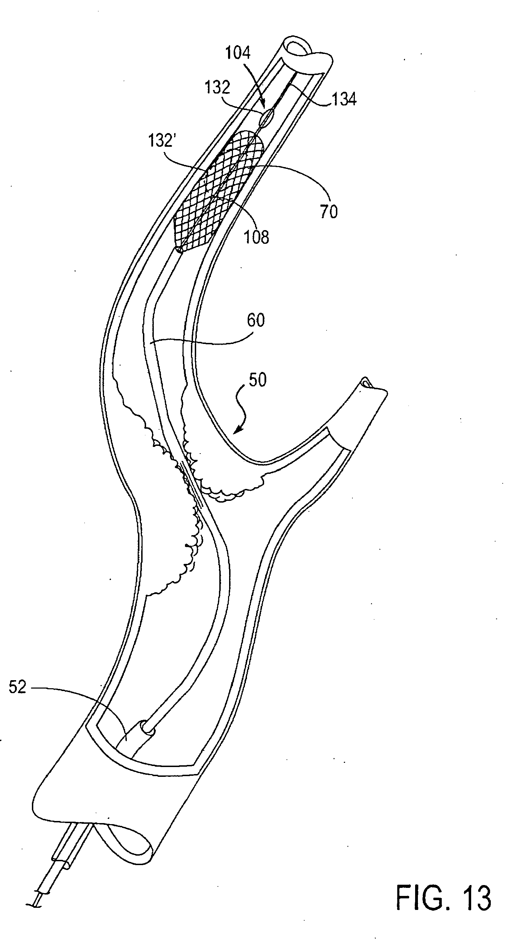

FIG 13 shows a stent delivery sheath 60 advanced across the stenosis 50 and deploying a self-expanding stent 70 within the lesion. The self-expanding stent 70 is deployed by pulling the stent delivery sheath 60 by the gripping portion 62 on its proximal end while maintaining the position of the combination angioplasty balloon catheter and stent pusher catheter 102. The stent 70 is typically deployed without embolic protection as this step presents very low risk for release of embolic material. Optionally however, the occlusion balloon 132 on the embolic protection device 104 can be inflated to provide embolic protection during this step of the procedure if desired. The occlusion balloon 132 can be inflated distally to the treatment site prior to release of the self-expanding stent 70 or, alternatively, the occlusion balloon 132 can be inflated at a distal location within the lumen of the self-expanding stent 70 after it has been partially deployed as indicated by the phantom lines 132' in FIG 13.

Most practitioners presently consider it very important to cover the whole atherosclerotic plaque with the stent from a normal arterial wall to a normal arterial wall. This implies the use of long stents. Because of the strong flow in the carotid artery there is no evidence, contrary to the experience in other arteries, that a long stent produces more restenosis than short stents at the carotid bifurcation. Alternatively, the catheter system 100 can be used for delivering shorter length stents, for example 3 cm or even shorter, where it is clinically indicated.

The recommended characteristics of the stent 70 for use in carotid bifurcations are: (a) the stent should be self-expanding, (b) preferably a minimum of 5 cm length should be used, (c) an expanded diameter of 7 to 9 mm is typically necessary to fit with the common carotid artery, (d) a good radial expansion force is mandatory to rule out secondary complications due to aggregation on poorly deployed stents, (e) continuous, not segmented, framework of the stent is recommended to get a straightening of the carotid artery that facilitates the stenting technique, (f) longer and conic stents might be considered in the future. These characteristics may be varied for adapting the stenting technique to other parts of the vasculature.

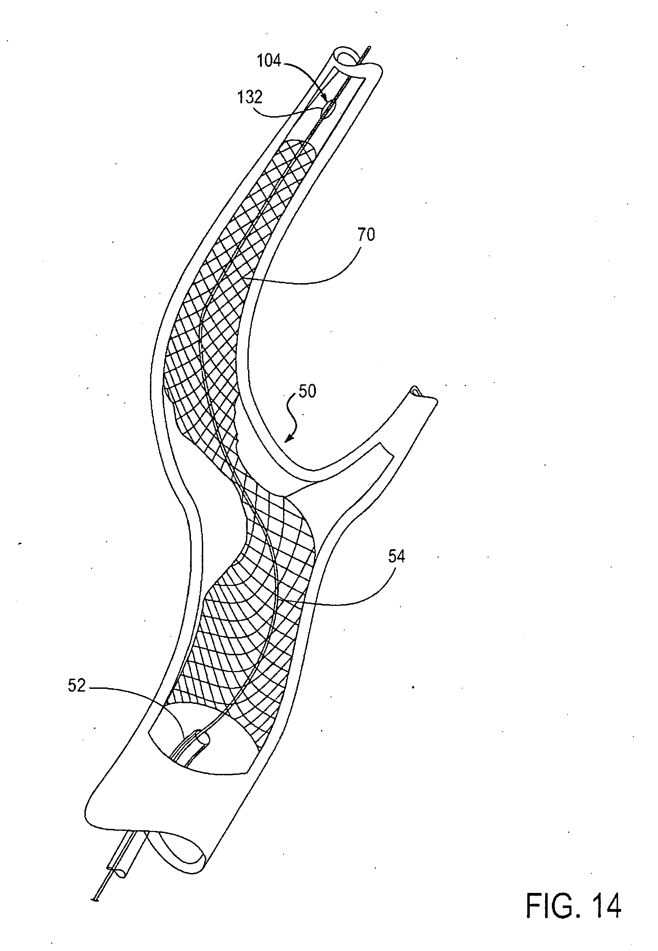

FIG 14 illustrates the self-expanding stent 70 deployed within the lesion. The stent delivery sheath 60 has been pulled back into the guiding catheter 52. A residual stenosis 50 may remain at the site of the original stenosis, but the entire length of the lesion is effectively covered by the expanded stent 70.

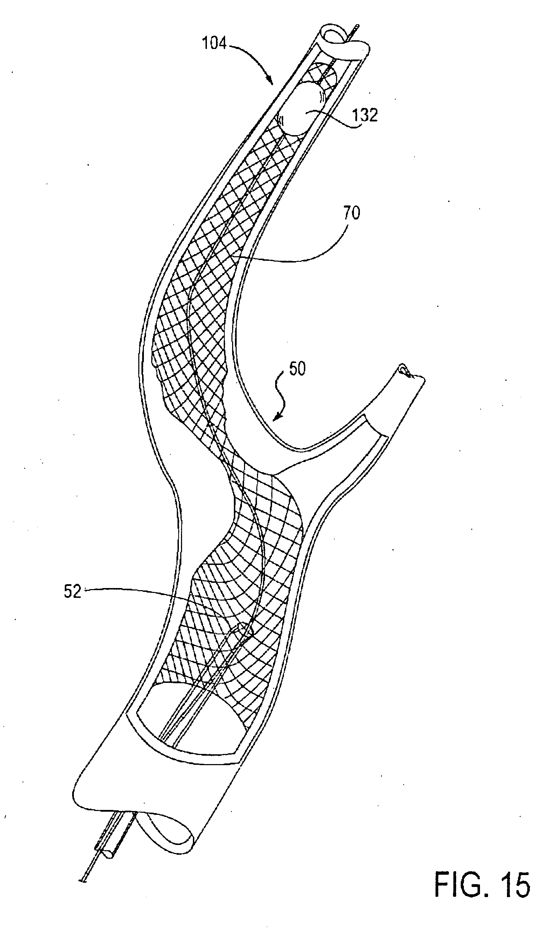

FIG 15 shows the distal end of the guiding catheter 52 advanced into the lumen of the deployed self expanding stent 70 and the occlusion balloon 132 of the embolic protection device 104 inflated within the lumen of the self-expanding stent. The occlusion balloon 132 is inflated in the distal part of the stent 70 to occlude the carotid artery and to prevent any embolic debris from traveling downstream from the treatment site. The guiding catheter 52 is firmly positioned into the lumen of the deployed self-expanding stent 70 leaving an open road for the following steps of the technique.

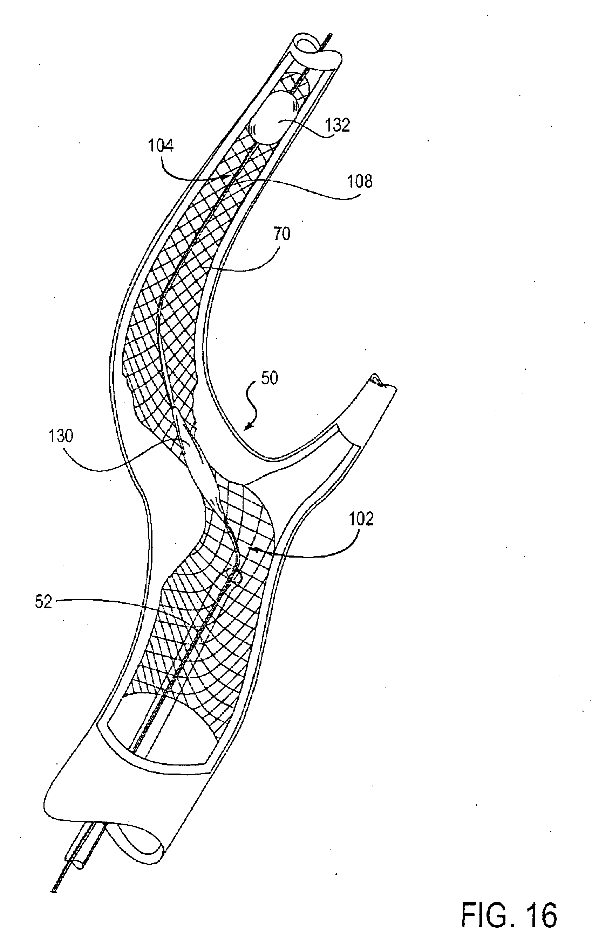

FIG 16 shows the combination angioplasty balloon catheter and stent pusher catheter 102 positioned with the angioplasty balloon 130 across the lesion 50.

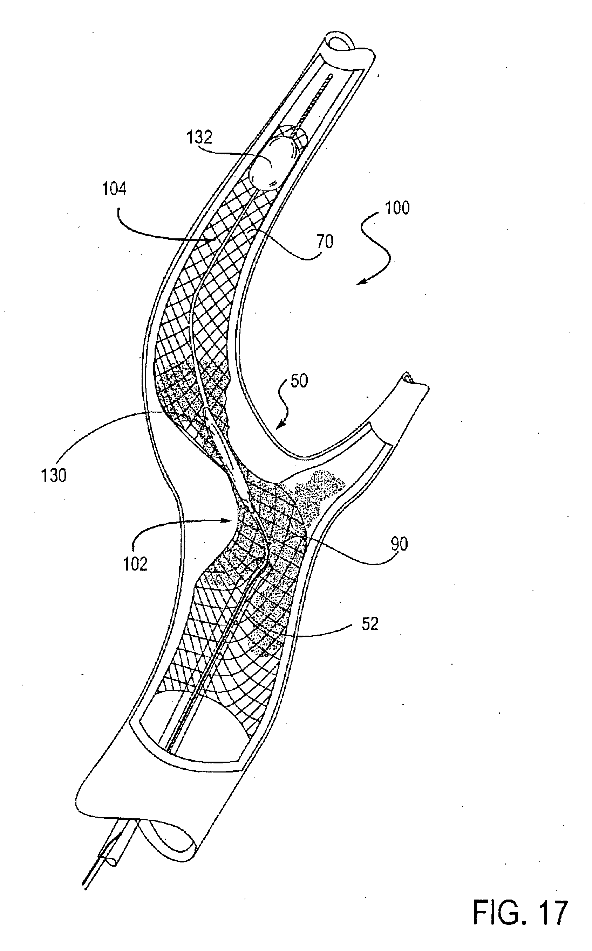

FIG 17 shows an angiography study performed to confirm occlusion of the internal carotid artery prior to dilatation of the lesion 50. The patient is clinically tested. An angiography series is performed to confirm the effective temporary occlusion of the internal carotid. The contrast 90 should remain close to the bifurcation site and usually does not reach the occlusion balloon 132.

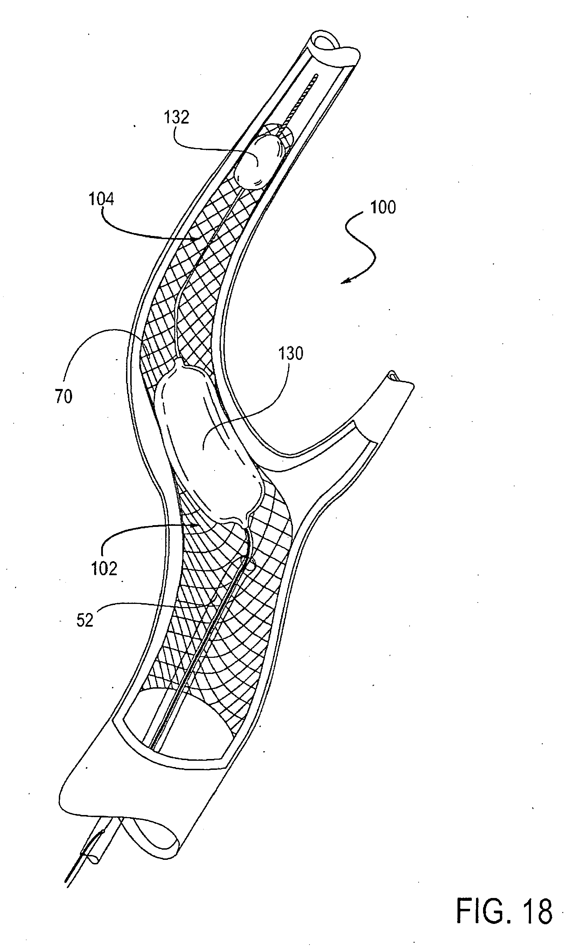

FIG 18 shows the angioplasty balloon 130 inflated to dilate the stenosis 50 and to complete the deployment or expansion of the self-expanding stent 70. It is recommended that atropine be injected at least 5 minutes previously to rule out bradycardia induced by the compression of the carotid glomus.

After completion of the poststenting angioplasty, the angioplasty balloon 130 is deflated and the combination angioplasty balloon catheter and stent pusher catheter 102 and the stent delivery sheath 60 are withdrawn from the guiding catheter 52. The tubular shaft 108 of the embolic protection device 104 has sufficient length that the distal section 112 of the combination angioplasty balloon catheter and stent pusher catheter 102 and the stent delivery sheath 60 can be "parked" on the shaft 108 near the proximal end of the embolic protection device 104 so that it will not interfere with the aspiration step, which is to follow. (In the case of the over-the-wire version of the catheter system 100 of FIG 8, the tubular shaft 108 of the embolic protection device 104 has additional length so that the full length of the combination angioplasty balloon catheter and stent pusher catheter 102 and the stent delivery sheath 60 can be "parked" on the shaft 108 near the proximal end of the embolic protection device 104.) Alternatively, if the embolic protection device 104 is made with a removable proximal fitting, the fitting may be removed at this point so that the angioplasty catheter 102 can be removed completely. The internal sealing member described above will maintain the occlusion balloon 132 in the inflated state.

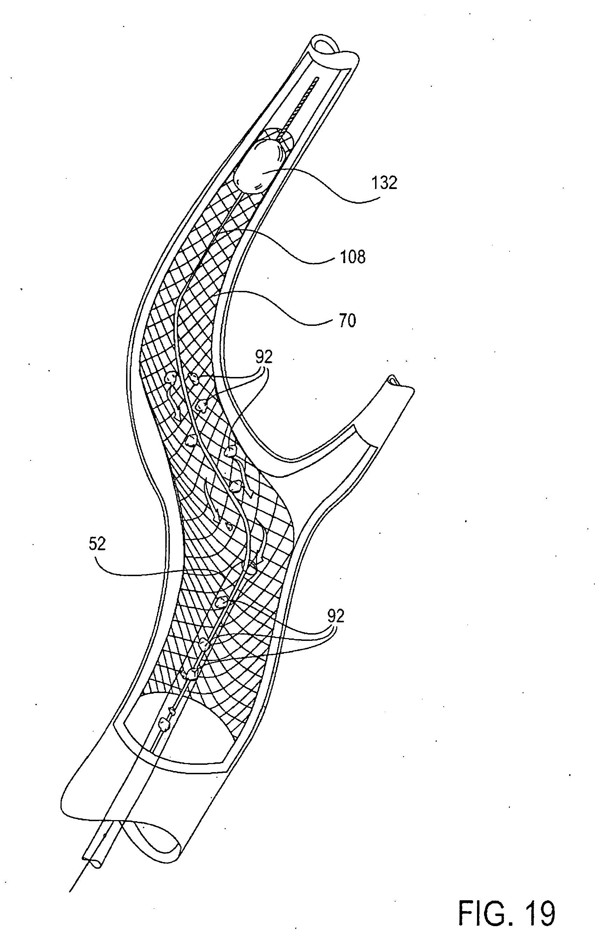

With the occlusion balloon 132 still inflated, blood is aspirated back through the lumen of the guiding catheter 52. FIG 19 illustrates potential embolic material 92 being aspirated through the lumen of the guiding catheter 52.

Alternatively, the stent delivery sheath 60 can be left in place and the lumen of the sheath 60 can be used for aspiration and/or irrigation of potential embolic material 92.

The occlusion balloon 132 is then deflated and the angioplasty catheter 102 and embolic protection device 104 are withdrawn. An angiography series is performed through the guiding catheter 52 to verify patency of the lumen and full deployment of the self-expanding stent 70. Then, the guiding catheter 52 and introducer are withdrawn and the puncture site is closed.

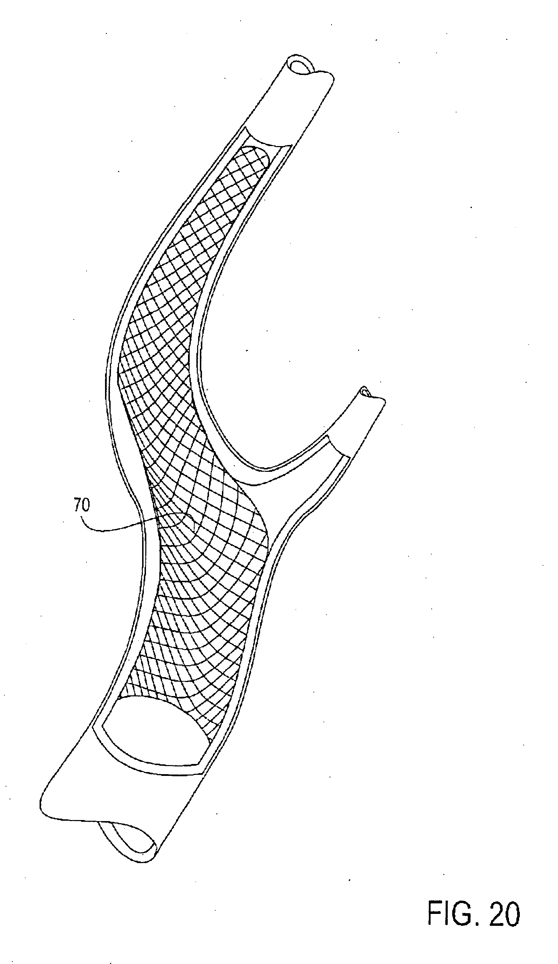

FIG 20 illustrates the patient's carotid bifurcation with the fully deployed stent 70 after completion of the protected angioplasty and stenting procedure.

Although it has been described in relation to treatment of obstructive carotid artery disease, the method of the present invention can be adapted for performing protected angioplasty and stenting in other parts of the vasculature, for example in the coronary arteries or renal arteries.

While the present invention has been described herein with respect to the exemplary embodiments and the best mode for practicing the invention, it will be apparent to one of ordinary skill in the art that many modifications, improvements and subcombinations of the various embodiments, adaptations and variations can be made to the invention without departing from the scope thereof.

## Claims

1. A catheter system for stenting and angioplasty, comprising:
a stent delivery sheath (60) having a proximal end (62) and a distal end and an internal lumen;
a self-expanding stent (70) having an unexpanded condition and an expanded condition;
and a combination angioplasty and stent pusher catheter (102) having a catheter shaft (106, 107) with an expandable member (130) mounted near a distal end of said catheter shaft;
wherein said catheter system has an undeployed configuration in which said self-expanding stent is in said unexpanded condition and is positioned within a distal portion of said internal lumen of said stent delivery sheath, and said combination angioplasty and stent pusher catheter is positioned within said internal lumen of said stent delivery sheath proximal to said self-expanding stent, whereby said combination angioplasty and stent pusher catheter can be used to deploy said self-expanding stent by retracting said stent delivery sheath while maintaining said combination angioplasty and stent pusher catheter to release said self-expanding stent out of said distal end of said stent delivery sheath thereby allowing said self-expanding stent to expand to said expanded condition, and subsequently advancing said combination angioplasty and stent pusher catheter distally until said expandable member is located within said self-expanding stent and expanding said expandable member to further expand said self-expanding stent.

2. The catheter system of claim 1, wherein said combination angioplasty and stent pusher catheter comprises a shoulder (142) located at a distal end of said combination angioplasty and stent pusher catheter, said shoulder sized and configured to push said self-expanding stent through said internal lumen of said stent delivery sheath.

3. The catheter system of claim 1 or 2, wherein said expandable member of said combination angioplasty and stent pusher catheter comprises an inflatable angioplasty balloon.

4. The catheter system of any of claims 1 to 3, further comprising an embolic protection device sized and configured to be insertable through said internal lumen of said stent delivery sheath.

5. The catheter system of claim 4, wherein said combination angioplasty and stent pusher catheter comprises a through lumen and said embolic protection device comprises a shaft sized and configured to be insertable through said through lumen of said combination angioplasty and stent pusher catheter and an inflatable occlusion balloon mounted near a distal end of said shaft.

6. The catheter system of claim 5, wherein, when said catheter system is in the undeployed configuration, said inflatable occlusion balloon is positioned at least partially within said internal lumen of said stent delivery sheath and distal to said self-expanding stent.

7. The catheter system of claim 6, wherein said combination angioplasty and stent pusher catheter is configured as a rapid exchange catheter such that said through lumen extends through said catheter shaft from said distal end to a proximal opening located on said catheter shaft at a location intermediate between said expandable member and a proximal end of said catheter shaft.

8. The catheter system of claim 7, wherein said stent delivery sheath has an opening through a wall of said stent delivery sheath communicating with said internal lumen at a location intermediate between said distal end and said proximal end of said stent delivery sheath, said opening being sized and configured to allow said shaft of said embolic protection device to be inserted therethrough.

9. The catheter system of claim 8, further comprising an auto-releasing sheath that surrounds a proximal section of said shaft of said embolic protection device and a proximal section of said stent delivery sheath to hold said embolic protection device and said stent delivery sheath together such that said catheter system can be inserted into a patient as a single unit.

10. The catheter system of claim 9, wherein said auto-releasing sheath has a tubular configuration with a longitudinal slit extending from a distal end to a proximal end of said auto-releasing sheath, and wherein said distal end of said auto-releasing sheath is cut at a diagonal with a tab located on the opposite side from said longitudinal slit.

11. The catheter system of claim 6, wherein said combination angioplasty and stent pusher catheter is configured as an over-the-wire catheter such that said through lumen extends through said catheter shaft from said distal end to a proximal end of said catheter shaft.

12. A catheter system according to any of the previous claims, wherein said stent delivery sheath comprises:
a hemostasis valve at said proximal end configured to provide a seal around said catheter shaft of said combination angioplasty and stent pusher catheter;
and a side fitting communicating with said internal lumen of said stent delivery sheath distal to said hemostasis valve.

13. The catheter system of claim 5, wherein said combination angioplasty and stent pusher catheter comprises a through lumen and said embolic protection device comprises a shaft sized and configured to be insertable through said through lumen of said combination angioplasty and stent pusher catheter and an expandable embolic protection filter mounted near a distal end of said shaft.

14. The catheter system of claim 13, wherein, when said catheter system is in the undeployed configuration, said expandable embolic protection filter is positioned at least partially within said internal lumen of said stent delivery sheath and distal to said self-expanding stent.

## Patentansprüche

1. Kathetersystem für Stenting und Angioplasie, umfassend:
eine Stentabgabehülle (60) mit einem proximalen Ende (62) und einem distalen Ende und einem inneren Lumen,
einen selbstexpandierenden Stent (70) mit einem unexpandierten Zustand und einem expandierten Zustand,
und einen kombinierten Angioplastie- und Stentschubkatheter (102), der einen Katheterschaft (106, 107) mit einem in der Nähe des distalen Endes des Katheterschafts angeordneten expandierbaren Element (130) umfaßt,
wobei das Kathetersystem eine unaufgefaltete Konfiguration besitzt, in welcher der selbstexpandierende Stent im unexpandierten Zustand ist und in einem distalen Abschnitt des inneren Lumens der Stentabgabehülle angeordnet ist und der kombinierte Angioplastie- und Stentschubkatheter in dem inneren Lumen der Stentabgabehülle proximal zum selbstexpandierenden Stent angeordnet ist,
wobei der kombinierte Angioplastie- und Stentschubkatheter benutzt werden kann, um den selbstexpandierenden Stent durch Zurückziehen der Stentabgabehülle abzugeben, während der kombinierte Angioplastie- und Stentschubkatheter gehalten wird, um den selbstexpandierbaren Stent aus dem distalen Ende der Stentabgabehülle freizugeben, wodurch die Expansion des selbstexpandierenden Stents zum expandierten Zustand erlaubt wird, und anschließend den kombinierten Angioplastie- und Stentschubkathether distal vorzuschieben, bis sich das expandierbare Element in dem selbstexpandierenden Stent befindet, und das expandierbare Element zu expandieren, um den selbstexpandierenden Stent weiter zu expandieren.

2. Kathetersystem nach Anspruch 1, wobei der kombinierte Angioplastie- und Stentschubkatheter eine Schulter (142) umfaßt, die an einem distalen Ende des kombinierten Angioplastie- und Stentschubkatheters angeordnet ist, wobei die Schulter bemaßt und konfiguriert ist, um den selbstexpandierenden Stent durch das innere Lumen der Stentabgabehülle zu schieben.

3. Kathetersystem nach einem der Ansprüche 1 oder 2, wobei das expandierbare Element des Angioplastie- und Stentschubkatheters einen aufblasbaren Angioplastieballon umfaßt.

4. Kathetersystem nach einem der Ansprüche 1 bis 3, ferner umfassend eine Embolieschutzvorrichtung, die bemaßt und konfiguriert ist, um durch das innere Lumen der Stentabgabehülle einführbar zu sein.

5. Kathetersystem nach Anspruch 4, wobei der kombinierte Angioplastie- und Stentschubkatheter ein durchgehendes Lumen umfaßt und die Embolieschutzvorrichtung einen Schaft, der bemaßt und konfiguriert ist, um durch das durchgehende Lumen des kombinierten Angioplastie- und Stentschubkatheters einsteckbar zu sein, und einen Verschlußballon, der in der Nähe des distalen Endes des Schaftes angeordnet ist, umfaßt.

6. Kathetersystem nach Anspruch 5, wobei, wenn das Kathetersystem in der unentfalteten Konfiguration ist, der aufblasbare Verschlußballon wenigstens teilweise in dem inneren Lumen der Stentabgabehülle und distal zum selbstexpandierenden Stent angeordnet ist.

7. Kathetersystem nach Anspruch 6, wobei der kombinierte Angioplastie- und Stentschubkathether als Schnellaustauschkatheter ausgebildet ist, so daß das durchgehende Lumen sich durch den Katheterschaft von dem distalen Ende bis zu einer proximalen Öffnung, die auf den Katheterschaft an einer zwischenliegenden Stelle zwischen dem expandierbaren Element und einem proximalen Ende des Katheterschaftes angeordnet ist, erstreckt.

8. Kathetersystem nach Anspruch 7, wobei die Stentabgabehülle an einer zwischenliegenden Stelle zwischen dem distalen Ende und dem proximalen Ende der Stentabgabehülle eine Öffnung durch eine Wand der Stentabgabehülle besitzt, die mit dem inneren Lumen verbunden ist, wobei die Öffnung so bemaßt und konfiguriert ist, daß der Schaft der Embolieschutzvorrichtung hindurchgeführt werden kann.

9. Kathetersystem nach Anspruch 8, ferner umfassend eine selbstfreigebende Hülle, die einen proximalen Abschnitt des Schaftes der Embolieschutzvorrichtung und einen proximalen Abschnitt der Stentabgabehülle umfaßt, um die Embolieschutzvorrichtung und die Stentabgabehülle so zusammenzuhalten, daß das Kathetersystem in einen Patienten als eine Einheit eingeführt werden kann.

10. Kathetersystem nach Anspruch 9, wobei die selbstfreigebende Hülle eine röhrenförmige Konfiguration mit einem länglichen Schlitz besitzt, der sich von einem distalen Ende bis zu einem proximalen Ende der selbstfreigebenden Hülle erstreckt, und wobei das distale Ende der selbstfreigebenden Hülle schräg abgeschnitten und mit einem tabulatorähnlichen Vorsprung an der dem Schlitz gegenüberliegenden Seite versehen ist.

11. Kathetersystem nach Anspruch 6, wobei der kombinierte Angioplastie- und Stentschubkatheter als drahtgeführter Katheter konfiguriert ist, so daß das durchgehende Lumen sich durch den Katheterschaft von dem distalen Ende bis zum proximalen Ende des Katheterschafts erstreckt.

12. Kathetersystem nach einem der vorhergehenden Ansprüche, wobei die Stentabgabehülle umfaßt:
ein hemostatisches Ventil an dem proximalen Ende, das dazu ausgebildet ist, eine Abdichtung um den Katheterschaft des kombinierten Angioplastie- und Stentschubkatheters bereitzustellen,
und einen distal von dem hemostatischen Ventil angeordneten Seitenanschluß, der mit dem inneren Lumen der Stentabgabehülle in Verbindung ist.

13. Kathetersystem nach Anspruch 5, wobei der kombinierte Angioplastie- und Stentschubkatheter ein durchgehendes Lumen umfaßt und die Embolieschutzvorrichtung einen Schaft, der bemaßt und konfiguriert ist, um durch das durchgehende Lumen des kombinierten Angioplastie- und Stentschubkatheters einführbar zu sein, und einem expandierbaren Embolieschutzfilter, der in der Nähe eines distalen Endes des Schaftes angeordnet ist, umfaßt.

14. Kathetersystem nach Anspruch 13, wobei, wenn das Kathetersystem in der unaufgefalteten Konfiguration ist, der expandierbare Embolieschutzfilter wenigstens teilweise in dem inneren Lumen der Stentabgabehülle distal zum selbstexpandierenden Stent angeordnet ist.

## Revendications

1. Système de cathéter pour la pose de stent et pour l'angioplastie, comprenant :
une gaine d'amenée de stent (60) ayant une extrémité proximale (62) et une extrémité distale et une lumière interne ;
un stent à autoexpansion (70) ayant une condition sans expansion et une condition en expansion ;
et une combinaison de cathéter d'angioplastie et de cathéter pousse-stent (102) ayant une tige de cathéter (106, 107) avec un élément expansible (130) monté à proximité d'une extrémité distale de ladite tige de cathéter ;
dans lequel ledit système de cathéter présente une configuration non déployée dans laquelle ledit stent à autoexpansion se trouve dans la condition sans expansion et est positionné dans une portion distale de ladite lumière interne de ladite gaine d'amenée de stent, et ladite combinaison de cathéter d'angioplastie et de cathéter pousse-stent est positionnée dans ladite lumière interne de ladite gaine d'amenée de stent à proximité dudit stent à autoexpansion, grâce à quoi ladite combinaison de cathéter d'angioplastie et de cathéter pousse-stent peut être utilisée pour déployer ledit stent à autoexpansion en rétractant ladite gaine d'amenée de stent tout en maintenant ladite combinaison de cathéter d'angioplastie et de cathéter pousse-stent pour relâcher ledit stent à autoexpansion hors de ladite extrémité distale de ladite gaine d'amenée de stent, permettant ainsi audit stent à autoexpansion de subir une expansion à ladite condition en expansion, et faire avancer ultérieurement ladite combinaison de cathéter d'angioplastie et de cathéter pousse-stent en direction distale jusqu'à ce que ledit élément expansible soit situé à intérieur dudit stent à autoexpansion et mettre en expansion ledit élément expansible pour poursuivre l'expansion dudit stent à autoexpansion.

2. Système de cathéter selon la revendication 1, dans lequel ladite combinaison de cathéter d'angioplastie et de cathéter pousse-stent comprend un épaulement (142) situé à une extrémité distale de ladite combinaison de cathéter d'angioplastie et de cathéter pousse-stent, ledit épaulement ayant une taille et une configuration propre à pousser ledit stent à autoexpansion à travers ladite lumière interne de ladite gaine d'amenée de stent.

3. Système de cathéter selon la revendication 1 ou 2, dans lequel ledit élément expansible de ladite combinaison de cathéter d'angioplastie et de cathéter pousse-stent comprend un ballon d'angioplastie gonflable.

4. Système de cathéter selon l'une quelconque des revendications 1 à 3, comprenant en outre un dispositif de protection embolique de taille et de configuration propre à être inséré à travers ladite lumière interne de ladite gaine d'amenée de stent.

5. Système de cathéter selon la revendication 4, dans lequel ladite combinaison de cathéter d'angioplastie et de cathéter pousse-stent comprend une lumière traversant et ledit dispositif de protection embolique comprend une tige d'une taille et d'une configuration propre à être insérée à travers ladite lumière traversant de ladite combinaison de cathéter d'angioplaste et de cathéter pousse-stent, et un ballon d'occlusion gonflable monté à proximité d'une extrémité distale de ladite tige.

6. Système de cathéter selon la revendication 5, dans lequel, quand ledit système de cathéter est dans la configuration non déployée, ledit ballon d'occlusion gonflable est positionné au moins partiellement dans ladite lumière interne de ladite gaine d'amenée de stent et en position distale par rapport audit stent à autoexpansion.

7. Système de cathéter selon la revendication 6, dans lequel ladite combinaison de cathéter d'angioplastie et de cathéter pousse-stent est configurée sous forme de cathéter à échange rapide de telle façon que ladite lumière traversant s'étend à travers ladite tige de cathéter depuis ladite extrémité distale jusqu'à une ouverture proximale située sur ladite tige de cathéter à un emplacement intermédiaire entre ledit élément expansible et une extrémité proximale de ladite tige de cathéter.

8. Système de cathéter selon la revendication 7, dans lequel ladite gaine d'amenée de stent présente une ouverture à travers une paroi de ladite gaine d'amenée de stent en communication avec ladite lumière interne à un emplacement intermédiaire entre ladite extrémité distale et ladite extrémité proximale de ladite gaine d'amenée de stent, ladite ouverture ayant une taille et une configuration propre à permettre à ladite tige dudit dispositif de protection embolique d'être insérée à travers celle-ci.

9. Système de cathéter selon la revendication 8, comprenant en outre une gaine à auto-relâchenient qui entoure une section proximale de ladite tige dudit dispositif de protection embolique et une section proximale de ladite gaine d'amenée de stent pour tenir ledit dispositif de protection embolique et ladite gaine d'amenée de stent ensemble, de sorte que ledit système de cathéter peut être inséré dans un patient sous forme d'une unité unique.

10. Système de cathéter selon la revendication 9, dans lequel ladite gaine à auto-relâchement présente une configuration tubulaire avec une fente longitudinale s'étendant depuis une extrémité distale jusqu'à l'extrémité proximale de ladite gaine à auto-relâchement, et dans lequel ladite extrémité distale de ladite gaine à auto-relâchement est coupée en diagonale avec une languette située sur le côté opposé depuis ladite fente longitudinale.

11. Système de cathéter selon la revendication 6, dans lequel ladite combinaison de cathéter d'angioplastie et de cathéter pousse-stent est configurée sous forme de cathéter coaxial sur fil (OTW) de telle façon que ladite lumière traversant s'étend à travers ladite tige de cathéter depuis ladite extrémité distale jusqu'à l'extrémité proximale de ladite tige de cathéter.

12. Système de cathéter selon l'une quelconque des revendications précédentes, dans lequel ladite gaine d'amenée de stent comprend :
une valve hémostatique à ladite extrémité proximale, configurée pour assurer un joint autour de ladite tige de cathéter de ladite combinaison de cathéter d'angioplastie et de cathéter pousse-stent ;
et un raccord latéral en communication avec ladite lumière interne de ladite gaine d'amenée de stent en position distale par rapport à ladite valve hémostatique.

13. Système de cathéter selon la revendication 5, dans lequel ladite combinaison de cathéter d'angioplastie et de cathéter pousse-stent comprend une lumière traversant et ledit dispositif de protection embolique comprend une tige d'une taille et d'une configuration propre à être insérée à travers ladite lumière traversant de ladite combinaison de cathéter d'angioplastie et de cathéter pousse-stent, et un filtre de protection embolique expansible monté à proximité d'une extrémité distale de ladite tige.

14. Système de cathéter selon la revendication 13, dans lequel, quand ledit système de cathéter est dans la configuration non déployée, ledit filtre de protection embolique expansible est positionné au moins partiellement dans ladite lumière interne de ladite gaine d'amenée de stent et en position distale par rapport audit stent à autoexpansion.
